# EUROPÄISCHE PATENTANMELDUNG

(11) **EP 1 435 378 A1**
(43) Veröffentlichungstag der Anmeldung: **07.07.2004**
(21) Anmeldenummer: 03029163.7
(22) Anmeldetag: 18.12.2003
(51) Int. Cl.: C09C 3/10, C09C 1/24

(54) **Funktionalisierung unreaktiver Substrate**

(30) Priorität: 20.12.2002 DE 10260918; 02.09.2003 DE 10340800
(71) Anmelder: MERCK PATENT GmbH, 64293 Darmstadt (DE)
(72) Erfinder: Nies, Berthold, Dr., 64407 Fränkisch-Crumbach (DE); Jeschke, Brigitte, Dr., 65779 Kelkheim (DE); Schilke, Frank, 64331 Weiterstadt (DE); Koch, Matthias, Dr., 65183 Wiesbaden (DE); Kübelbeck, Armin, 64625 Bensheim (DE)

(57) **Zusammenfassung**

Die Erfindung beschreibt ein Verfahren zur Herstellung von funktionalisierten unreaktiven Substraten, wobei die Oberfläche des Substrates zunächst mit einem Melamin-Formaldehydharz beschichtet und anschließend mit einem Funktionalisierungsreagenz umgesetzt wird.

## Beschreibung

Gegenstand der Erfindung ist ein Verfahren zur Herstellung funktionalisierter Substrate sowie ein anorganischer oder organischer Formkörper.
Das erfindungsgemäße Verfahren ist eine Parallelentwicklung des, in der DE 10260918.7 ("Knochenzement mit verbesserten mechanischen Eigenschaften und Verfahren zu seiner Herstellung") beschriebenen Verfahrens und beansprucht deren Priorität. Desweiteren ist das erfindungsgemäße Verfahren eine Weiterentwicklung des in der DE 102 09 359.8 beschriebenen Verfahrens.

Kompositmaterialien kommen in vielen Bereichen der Technik zur Anwendung. Beispiele sind Pigmente in Lacken, Füllstoffe zur Verbesserung der Materialeigenschaften von Kunststoffen oder Dispersionen fester Teilchen in wässrigen oder nicht wässrigen Flüssigkeiten. Hierbei müssen oft chemisch und physikalisch unterschiedlichen Materialien miteinander gemischt werden. Eine homogene Verteilung wird meist nur durch Kompatibilisierung des Füllmateriales erreicht. Zum Beispiel lassen sich die Eigenschaften von Siliziumdioxidoberflächen (Gläser) wie dem Fachmann bekannt, durch Reaktion mit entsprechenden Silanen verändern.

Diese Funktionalisierung setzt jedoch eine gewisse Reaktivität der Oberfläche voraus, welche bei vielen Substraten nicht gegeben ist. Anorganische Pigmente auf Metalloxidbasis sind mit dieser Methode auf Grund mangelnder Reaktivität oftmals nicht veränderbar.

Aufgabe der vorliegenden Erfindung war es daher, ein Verfahren zu entwickeln, welches unreaktive Substrate durch spezielle Beschichtungen ausreichend aktiviert um die Substrate anschließend zu funktionalisieren.

Diese Aufgabe wird gelöst durch ein Verfahren zur Herstellung funktionalisierter (unreaktiver) Substrate, wobei die Oberfläche des Substrates zunächst mit einem Melamin-Formaldehydharz als reaktive Zwischenschicht beschichtet und anschließend mit einem Funktionalisierungsreagenz umgesetzt wird.

Unreaktive Substrate bedeutet, dass sich diese Substrate nicht oder nur schlecht funktionalisieren lassen. Als Substrate kommen anorganische oder organische Formkörper beliebiger Geometrie für Kunststoffe, Metalle, Glas, Keramik etc. in Frage, wobei ein partikuläres Material bevorzugt ist. Noch bevorzugter sind Substrate, die als Pigmente oder Füllstoffe für Kunststoffmaterialien Anwendung finden.

Als reaktive Zwischenschicht kommt Melamin-Formaldehydharz (kommerziell erhältlich z.B. als Madurit® von der Firma Solutia GmbH, Wiesbaden) in Betracht, das sich leicht auf beliebige Substrate aufbringen lässt. Durch säurekatalysierte dreidimensionale Vernetzung entsteht ein fester Belag, der auch ohne chemische Bindung zum Substrat fest auf diesem haftet. In DE 102 09 359.8 ist dieses Verfahren ausführlich beschrieben. Nach dem in DE 102 10 779.3 beschriebenen Verfahren kann die Beschichtung mit Melamin-Formaldehyd-Harz an Stelle von SäureZugabe, durch den Zusatz von Wasserstoffperoxid-Lösung eingeleitet werden.
Die Melamin-Formaldehydharz-Schicht erlaubt die Funktionalisierung mit einer Reihe handelsüblicher, verglichen mit Silanen, preiswerter Reagenzien und damit eine effiziente, kostengünstige Kompatibilisierung unterschiedlichster Materialien. Weiterhin lassen sich so funktionalisierte Teilchen als Trägermaterialien zur Immobilisierung einsetzen.

Bevorzugt besteht das Verfahren aus folgenden Schritten:
a) Suspendieren des Substrates in Wasser
b) Zugabe von Melamin-Formaldehydharz
c) Zugabe einer Säure, vorzugsweise Ameisensäure oder p-Toluolsulfonsäure
d) Waschen und Trocknen des Melamin-Formaldehydharzbeschichteten Substrates
e) Umsetzung mit einem Funktionalisierungsreagenzes, vorzugsweise mit einem Säurechlorid, Säureanhydrid, Säureazid, Aktivester, Aldehyd oder Ester.
f) Waschen und Trocknen des funktionalisierten Substrates.

Über die Funktionalisierung hinaus ist erfindungsgemäß die Herstellung von Kern-Mantel Partikel in folgenden Schritten möglich:
a) Suspendieren der unreaktiven Partikel in Wasser und anschließendem Erhitzen
b) Zugabe von Melamin-Formaldehydharz
c) Zugabe einer Säure wie z.B. Ameisensäure
d) Waschen und Trocknen der mit Melamin-Formaldehydharzbeschichteten Partikel
e) Zugabe eines Säurehalogenids als Funktionalisierungsreagenz bei Raumtemperatur unter Rühren
f) Waschen und Trocknen der funktionalisierten Partikel
g) Zugabe eines Metallhalogenids (z.B. Kuperbromid), eines Metallkomplexierungsreagenzes (z.B. 4,4'-di-(5-nonyl)-2,2'-bipyridin) und eines radikalisch polymerisierbaren Monomers wie Styrol sowie Suspendieren in Toluol unter anschließendem Erhitzen
h) Waschen mit Alkohol und Trocknung der Partikel.

Das Aufbringen der Startergruppen erfolgt über die erfindungsgemäße Funktionalisierungsmethode über Säurehalogenide auf die Kerne nach der bekannten ATRP-Technik (Atom Transfer Radical Polymerisation) oder verwandte Verfahren. Prinzipiell geeignet sind alle gängigen, kontrolliert kettenaufbauenden Polymerisationsverfahren (radikalisch, anionisch, kationisch oder metallkatalysiert) nach Funktionalisierung des Substrates mit den entsprechenden Startergruppen.

Weiterhin bevorzugt ist ein Verfahren zur Herstellung von Kern-Mantel Partikeln, wobei auf das mit Melamin-Formaldehydharzbeschichtete Substrat durch Polymerisation einer Epoxyverbindung wie Ethylenoxid eine Polymerschicht wie Polyethylenoxid aufgebracht wird.

Die Melaminoberfläche verfügt über reaktive Methylolgruppen, die sich besonders gut mit Säurechloriden, Säureanhydriden und Estern umsetzen lassen. Aber auch Säureazid, Aktivester oder Aldehyde sind geeignet. Je nach den gewünschten Oberflächeneigenschaften sind folgende Funktionalisierungen denkbar:
■ Hydrophobisierung mit langkettigen Alkylresten in Form von Estern aliphatischer Säuren.
■ Lipophobisierung mit perfluorierten Resten.
■ Ester von Acrylsäuren oder Vinylbenzoesäuren zum radikalischen Einpolymerisieren der Substrate in Polymermatrices.
■ Alkenylether zum Einpolymerisieren nach Ziegler-Natta und verwandten Verfahren.
■ Veresterung mit Isonicotinoylchlorid oder ähnlichen Verbindungen zur Erzeugung einer basischen Oberfläche
■ Hydrophilisierung mit Polyethylenoxid (Grafting from: Aufbau der Polymerkette vom Substrat weg),
■ Ester von Isobuttersäurehalogeniden und verwandten Verbindungen als ATRP-Initiator; Erzeugung definierter Polymerschichten mittels radikalischer Polymerisation (Core-Shell-Partikel)

### Beispiele:

1. Funktionalisierung eines Eisenpigmentes mit Laurinsäurechlorid:
   300 g Eisenpigment Iriodin 504 ® (Merck) werden in 5 I Wasser aufgeschlämmt und auf 70 C temperiert. Hierzu kommen unter Rühren 225 g Madurit® SMW 818 75% (Melamin-Formaldehydharz von Solutia Germany). Nachdem alles Madurit® gelöst ist, werden in einem Schwung 500 ml 2%ige Ameisensäure zugegeben und für 30 Min. gerührt. Das Produkt wird abgesaugt und mit Wasser gewaschen.
   100g des so beschichteten Eisenpigmentes werden in 250 ml Laurinsäurechlorid über Nacht am Rückfluss gekocht. Das Produkt wird abgesaugt und mit Wasser gewaschen. Man erhält ein hydrophobisertes Pigment.
2. Funktionalisierung von Zirkoniumoxid mit Methacrylsäureanhydrid:
   100g Zirkoniumoxid (Selectipur, Merck) werden in 5 I Wasser aufgeschlämmt und auf 70 C temperiert. Hierzu kommen unter Rühren 150 g Madurit MW 909 (Solutia Germany). Nachdem alles Madurit® gelöst ist, werden in einem Schwung 300 ml 2%ige Ameisensäure zugegeben und für 30 Min. gerührt. Das Produkt wird abgesaugt und mit Wasser gewaschen.
   10g des so beschichteten Zirkoniumoxids werden in 50 ml Methacrylsäureanhydrid (enthaltend 2% Hydroxychinonmonomethylether) aufgeschlämmt und über Nacht am Rückfluss gekocht. Das Produkt wird abgesaugt und mit Ethanol gewaschen. Man erhält einpolymerisierbares Zirkoniumoxid.
3. Funktionalisierung von Zirkoniumoxid mit Essigsäurechlorid:
   50g Zirkoniumoxid (Partikeldurchmesser ca. 50 nm) werden in 10 I Wasser aufgeschlämmt und auf 70 C temperiert. Hierzu kommen unter Rühren 100g Madurit MW 909 (Solutia Germany). Nachdem alles Madurit® gelöst ist, werden in einem Schwung 200 ml 10%ige Ameisensäure zugegeben und für 30 Min. gerührt. Das Produkt wird abzentrifugiert und mit Wasser gewaschen.
   10g des so beschichteten Zirkoniumoxids werden in 50 ml Acetylchlorid über Nacht bei Raumtemperatur gerührt. Das Produkt wird abgesaugt und mit Ethanol gewaschen. Man erhält hydrophobisiertes, nanoskaliges Zirkoniumchlorid.
4. Herstellung eines Kern-Mantel-Partikels durch Funktionalisierung mit Bromisobuttersäurebromid:
   5 g Melamin-Formaldehydharzbeschichtete Partikel werden über Nacht bei Raumtemperatur in 15 ml Bromisobuttersäurebromid gerührt. Die Partikel werden abgesaugt, mit Ethanol gewaschen und getrocknet.
   1 g dieser funktionalisierten Teilchen werden zusammen mit 75 mg CuBr, 400 mg 4,4'-di-(5-nonyl)-2,2'-bipyridin (= Metallkomplexierungsreagenz) und 7.5 g Styrol unter kräftigem Rühren in 50 ml Toluol suspendiert und auf 100 °C erwärmt. Die Kern-Mantel-Partikel werden mit Methanol gewaschen und getrocknet. Man erhält ein farbloses, leichtes Pulver.

## Patentansprüche

1. Verfahren zur Herstellung funktionalisierter Substrate, **dadurch gekennzeichnet, dass** die Oberfläche des Substrates zunächst mit einem Melamin-Formaldehydharz beschichtet und anschließend mit einem Funktionalisierungsreagenz umgesetzt wird.

2. Verfahren nach Anspruch 1, **dadurch gekennzeichnet, dass** die Herstellung funktionalisierter Substrate folgende Schritte umfasst:
a) Suspendieren des Substrates in Wasser
b) Zugabe von Melamin-Formaldehydharz
c) Zugabe einer Säure
d) Waschen und Trocknen des Melamin-Formaldehydharzbeschichteten Substrates
e) Umsetzung mit einem Funktionalisierungsreagenzes
f) Waschen und Trocknen des funktionalisierten Substrates

3. Verfahren nach den Ansprüchen 1 und/oder 2, **dadurch gekennzeichnet, dass** als Substrat ein chemisch unreaktives Substrat eingesetzt wird.

4. Verfahren nach Anspruch 3, **dadurch gekennzeichnet, dass** als unreaktives Substrat ein partikuläres anorganisches oder organisches Material verwendet wird.

5. Verfahren nach Anspruch 3, **dadurch gekennzeichnet, dass** als unreaktives Substrat ein Pigment oder Füllstoff für Kunststoffmaterialien verwendet wird.

6. Verfahren nach einem der Ansprüche 1 bis 5, **dadurch gekennzeichnet, dass** als Funktionalisierungsreagenz ein Säurechlorid, Säureanhydrid, Säureazid, Aktivester, Aldehyd oder Ester verwendet wird.

7. Verfahren zur Herstellung von Kern-Mantel Partikel aus einem unreaktiven Substrat, **dadurch gekennzeichnet, dass** die Herstellung folgende Schritte umfasst:
a) Suspendieren der unreaktiven Partikel in Wasser und anschließendem Erhitzen
b) Zugabe von Melamin-Formaldehydharz
c) Zugabe einer Säure
d) Waschen und Trocknen der mit Melamin-Formaldehydharzbeschichteten Partikel
e) Zugabe eines Säurehalogenids als Funktionalisierungsreagenz bei Raumtemperatur unter Rühren
f) Waschen und Trocknen der funktionalisierten Partikel
g) Zugabe eines Metallhalogenids, eines Metallkomplexierungsreagenzes und eines radikalisch polymerisierbaren Monomers sowie Suspendieren in Toluol unter anschließendem Erhitzen.
h) Waschen und Trocknen der Partikel.

8. Verfahren zur Herstellung von Kern-Mantel-Partikeln nach Anspruch 7, **dadurch gekennzeichnet, dass** auf das mit Melamin-Formaldehydharzbeschichtete Substrat durch Polymerisation einer Epoxyverbindung wie Ethylenoxid eine Polymerschicht wie Polyethylenoxid aufgebracht wird.

9. Anorganischer oder organischer Formkörper, erhältlich durch das Verfahren nach einem der Ansprüche 1 bis 8.

10. Anorganischer oder organischer Formkörper nach Anspruch 9, **dadurch gekennzeichnet, dass** er ein partikuläres Substrat ist.

11. Anorganischer oder organischer Formkörper nach Anspruch 9 und/oder 10, **dadurch gekennzeichnet, dass** er ein Pigment oder Füllstoff für Kunststoffmaterialien ist.
